# EUROPÄISCHE PATENTSCHRIFT

(11) **EP 0 294 711 B1**
(45) Veröffentlichungstag und Bekanntmachung des Hinweises auf die Patenterteilung: **10.03.1993**
(21) Anmeldenummer: 88108850.4
(22) Anmeldetag: 03.06.1988
(51) Int. Cl.: C12P 19/24, C12N 11/14

(54) **Verfahren zur Herstellung trägergebundener Enzyme**
Process for the preparation of enzymes fixed to a carrier
Procédé de préparation d'enzymes fixées sur un support

(30) Priorität: 10.06.1987 DE 3719324
(43) Veröffentlichungstag der Anmeldung: 14.12.1988
(73) Patentinhaber: Kali-Chemie Aktiengesellschaft, D-30173 Hannover (DE)
(72) Erfinder: Mücke, Ingo, D-3013 Barsinghausen (DE)
(74) Vertreter: Lauer, Dieter, Dr.

(56) Entgegenhaltungen:
- DE-A- 3 224 024
- DE-B- 2 726 188
- FR-A- 2 530 657

## Beschreibung

Die Erfindung betrifft ein Verfahren zur Herstellung trägergebundener Enzyme, bei dem man einen Träger, der zur Ausbildung von kovalenten Bindungen befähigte funktionelle Gruppen aufweist, mit einem Enzym belegt.

Bei der Durchführung enzymkatalysierter Reaktionen stehen als grundsätzliche Alternativen homogen katalysierte Verfahren unter Verwendung freier Enzyme und heterogen katalysierte Verfahren unter Verwendung trägergebundener Enzyme zur Wahl. Gegenüber freien Enzymen besitzen trägergebundene Enzyme vielfältige Vorteile, von denen vor allem ihre leichtere Wiederverwendbarkeit und ihre hervorragende Eignung für kontinuierlich durchzuführende Verfahren zu nennen sind. Als zu verwendende Träger kommen sowohl Träger aus organischen als auch aus anorganischen Materialien in Betracht. Dabei haben insbesondere bei kontinuierlichen, in industriellem Maßstab durchzuführenden Prozessen, in denen die trägergebundenen Enzyme in säulenförmigen Reaktoren in entsprechender Schütthöhe eingesetzt werden, die anorganischen Träger den Vorteil der geringen Kompressibilität. Während nun aber organische Träger häufig von Hause aus funktionelle Gruppen aufweisen, an die die Enzyme gebunden werden können, besitzen anorganische Träger solche funktionellen Gruppe in aller Regel nicht. Anorganische Träger müssen daher in einem gesonderten Schritt mit solchen funktionellen Gruppen versehen werden. Als Mittel der Wahl hat sich heute dafür die Behandlung mit Silane mit anorganisch funktionellen und organisch funktionellen Resten durchgesetzt. Klassischer Vertreter solcher Silane ist das gamma-Aminopropyltriethoxy-Silan, bei dem über die Hydrolyse der Ethoxy-Gruppen eine Si-O-Bindung zum Träger hergestellt wird, während die Aminopropyl-Gruppe als organisch funktioneller Rest zur Knüpfung von Bindungen in Richtung auf das Enzym zur Verfügung steht.

So schlägt beispielsweise die DE-1944418-B2 als grundlegende Literaturstelle für eine Verfahren der eingangs genannten Art vor, anorganische Träger mit Silanen der erwähnten Art zu behandeln und anschließend die Enzyme an den so funktionalisierten Träger, dort Aminoalkylsilanderivat genannt, entweder unmittelbar oder unter Zwischenschaltung eines "spacer" zu binden. Als geeignete anorganische Träger sind dort nichtsiliciumhaltige Metalloxide wie z.B. Aluminiumoxid, Hydroxylapatit oder Nickeloxid, und siliciumhaltige Substanzen wie poröses Glas, Kieselerde, Wollastonit, Silicagel oder Bentonit genannt.

Während der DE-1944418-B2 mit der Offenbarung des eingangs genannten Verfahrens das Verdienst zukommt, gezeigt zu haben, daß und wie anorganische Träger zur Herstellung trägergebundener Enzyme genutzt werden können, entwickelt die DE-2726188-C2 (≙ EP-28-B1) das eingangs genannte Verfahren weiter, indem diese Literaturstelle angibt, wie unter mehreren in Betracht kommenden Trägern derjenige Träger ermittelt werden kann, der hinsichtlich seiner Porengröße und Porenverteilung optimal ist, und wie die Konzentration der Enzymlösung, mit der der funktionalisierte Träger umgesetzt werden soll, beschaffen sein muß, um bei minimalem Enzym-Einsatz ein maximal aktives Präparat zu erhalten, das sich insbesondere auch dadurch auszeichnet, daß die Aktivität des trägergebundenen Enzyms gleich oder nur wenig kleiner ist als die Aktivität des Enzyms im freien Zustand.

Obgleich mit dem Verfahren gemäß der DE-2726188-C2 ausgezeichnete trägergebundene Enzyme mit hoher Ausgangsaktivität hergestellt werden können, so leiden nicht nur jene sondern grundsätzlich alle trägergebundenen Enzyme an einer nur begrenzten Stabilität, d.h. die Aktivität des trägergebundenen Enzyms nimmt unter den Einflüssen des praktischen Betriebs mehr oder weniger schnell ab.

Diesem Nachteil begegnet die DE-3148603-C1 (≙ EP-81185-B1) bei einem auf der Basis Si0₂-Träger hergestellten trägergebundenen Enzym dadurch, daß das Substrat vor der Umsetzung an dem trägergebundenen Enzym mit Formlingen aus Si0₂ oder Alumosilikat kontaktiert wird. Alternativ schlägt die DE-3405035-C1 (≙ EP-152036-A2) bei einem hinsichtlich des Trägers gleichartigen trägergebundenen Enzym vor, dem Substrat wasserlösliches Silikat zuzusetzen. Die beiden letztgenannten Verbesserungen sind jedoch auf trägergebundene Enzyme beschränkt, deren Träger ganz oder zu einem wesentlichen Teil aus kieselsäurehaltigem Material bestehen.

Aufgabe der Erfindung ist es daher, ein weiteres Verfahren zur Herstellung trägergebundenen Enzyme bereitzustellen, das auch bei solchen trägergebundenen Enzymen einsetzbar ist, deren Träger aus anderem als kieselsäurehaltigem Material besteht.

Dies gelingt erfindungsgemäß dadurch, daß man das trägergebundene Enzym zusätzlich in beliebiger Reihenfolge mit einem bifunktionellen Vernetzungsmittel und einem Polyamin behandelt.

Unter bifunktionellen Vernetzungsmitteln sind Substanzen wie Glutardialdehyd, Toluoldiisocyanat, Hexamethylendiisocyanat usw. zu verstehen. Die Erfindung bevorzugt Glutardialdehyd. Unter Polyaminen sind wasserlösliche Substanzen wie Ethylendiamin, Diethylentriamin, Triethylentetramin, Tetraethylenpentamin, Pentaethylenhexamin, Hexamethylendiamin, Polyethylenimin usw. zu verstehen. Die Erfindung bevorzugt Polyethylenimin, auch Polyethylenimin mit verzweigter Kette, mit einem Molekulargewicht bis zu 2.000.000 Dalton, vorzugsweise mit einem Molekulargewicht zwischen 600 und 1.000.000 Dalton.

Mit der erfindungsgemäßen zusätzlichen Behandlung mit dem bifunktionellen Vernetzungsmittel und dem Polyamin, in Sonderheit mit Glutardialdehyd und Polyethylenimin, lassen sich erheblich stabilere trägergebundene Enzyme herstellen als nach bekannten Verfahren.

Zwar ist die Verwendung von Glutardialdehyd und Polyethylenimin bei der Herstellung trägergebundener Enzyme an sich schon bekannt. So beschreibt die DE-2605797-B2 (≙ US-4141857-A) die Behandlung anorganischer poröser Träger mit Polyethylenimin und Glutardialdehyd, um ein, wie es dort heißt, kombiniertes organisch-anorganisches Grundmaterial aus einem anorganischen porösen Träger mit einem in dessen Poren adsorbierten und eingeschlossenen organischen Polymermaterial zu erzeugen. An das organische Polymermaterial wird dann in einem abschließenden Schritt das Enzym gebunden.

Die Behandlung des Trägers mit Polyethylenimin und Glutardialdehyd entspricht also der obenerwähnten Silanisierung, indem quasi zwischen dem Träger und dem Enzym eine Zwischenschicht erzeugt wird, die einerseits die Bindung zu dem Träger - bei der Silanisierung über Siloxanbildung, bei der Behandlung mit Polyethylenimin und Glutardialdehyd durch Formschluß des in den Poren des Trägers erzeigten Polymers - und andererseits die Bindung zum Enzym herzustellen im Stande ist. Mit anderen Worten, das durch die Behandlung poröser anorganischer Träger mit Polyethylenimin und Glutardialdehyd erhaltene "kombinierte organisch-anorganische Grundmaterial" entspricht dem im gattungsgemäßen Verfahren definierten "Träger, der zur Ausbildung von kovalenten Bindungen befähigte funktionelle Gruppen aufweist".

Während nun bei der DE-2605797-B2 die Behandlung des Trägers mit Polyethylenimin und Glutardialdehyd vor der Umsetzung mit dem Enzym stattfindet, findet demgegenüber bei der Erfindung die Behandlung mit Polyethylenimin und Glutardialdehyd als zusätzliche Maßnahme erst dann statt, wenn das Enzym schon an den Träger gebunden ist.

Das zur DE-2605797-B2 Gesagte trifft auch für die US-4438196-A zu, bei der zur Erzeugung eines Produkts, das dem kombinierten organisch-anorganischen Grundmaterial der DE-2605797-B2 entspricht, Aktivkohle mit Polyethylenimin und Glutardialdehyd behandelt wird.

Die ER 2 530 657 A1 beschreibt ein verfahren zur Herstellung trägergebundener biologischer Substanzen (wozu auch Enzyme gerechnet werden), bei dem ein Enzym an den Träger Vermiculit absorbiert wird, das erhaltene Vermiculit-Adsorbat zunächst mit einem Polyamin und sodann mit einem bifunktionellen Vernetzungsmittel behandelt wird. Um einen Träger, der im Sinne der vorliegenden Erfindung zur Ausbildung von kovalenten Bindungen befähigte funktionelle Gruppen aufweist, handelt es sich bei dem dort verwendeten Vermiculit nicht.

Noch weiter ab liegen Polyethylenimin benutzende Verfahren, repräsentiert beispielsweise durch die EP-133531-B1, bei denen Polyethylenimin, gegebenenfalls in Verbindung mit anderen Substanzen, dazu benutzt wird, um in Wasser gelöste Enzyme oder in Wasser suspendierte, intrazelluläre Enzyme enthaltende Mikroorganismenzellen auszuflocken. Das ausgeflockte Material wird dann in weiteren Schritten zu immobilisierten Biokatalysatoren konfektioniert.

Die erfindungsgemäße Behandlung mit Glutardialdehyd und Polyethylenimin kann in beliebiger Reihenfolge vorgenommen werden.

Bei der Behandlung mit Glutardialdehyd werden pro Gramm Katalysator-Trockensubstanz 5 bis 500 mg, vorzugsweise 25 bis 250 mg, Glutardialdehyd eingesetzt. Unter Katalysator-Trockensubstanz ist dabei das Gewicht des mit Silan behandelten Trägers einschließlich des an den Träger gebundenen Enyzms zu verstehen. Da im Verhältnis zur Masse des Träger die Massen des an ihn gebundenen Silan und des an ihn gebundenen Enzyms nur Bruchteile ausmachen, kann in erster Näherung die Katalysator-Trockensubstanz der Träger-Masse gleichgesetzt werden. Vorteilhafterweise erfolgt die Behandlung mit einer wäßrigen Lösung von Glutardialdehyd mit einer Konzentration (w/v) von 0,1 bis 10 %, vorzugsweise 0,5 bis 5 %, bei einem pH von 4 bis 9, vorzugsweise 5 bis 8, während einer Dauer von 15 bis 120 Minuten, vorzugsweise 30 bis 60 Minuten.

Die Behandlung mit Polyethylenimin erfolgt zweckmäßigerweise mit einer wäßrigen Lösung von Polyethylenimin mit einer Konzentration (w/v) von 0,1 bis 20 %, vorzugsweise 0,5 bis 8 %. Dabei werden vorteilhafterweise 3 bis 600, vorzugsweise 15 bis 240 mg Polyethylenimin pro Gramm Katalysator-Trockensubstanz eingesetzt. Zweckmäßigerweise wird bei der Behandlung ein pH von 4 bis 9, vorzugsweise 5 bis 8, eingestellt. Die Dauer der Behandlung erstreckt sich sinnvollerweise über wenigstens 5 Minuten, vorzugswiese über wenigstens 15 Minuten. Die zeitliche Obergrenze der Behandlung mit Polyethylenimin richtet sich nach der Menge des pro Gramm Katalysator-Trockensubstanz eingesetzten Polyethylenimin und der Konzentration der verwendeten Polyethyleniminlösung. Erfahrungsgemäß reicht ein Zeitraum von bis zu 24 Stunden aus, wobei ein befriedigendes Ergebnis bereits nach 12 Stunden erreicht ist. Eine längere Behandlung als 24 Stunden bringt keinen besonderen Effekt mehr.

Prinzipiell sind als Träger für das erfindungsgemäße Verfahren alle auch sonst gebräuchlichen Träger verwendbar. Die Erfindung bevorzugt die porösen anorganischen Träger auf Metalloxid- oder Silikat-Basis. Als typische Vertreter seine Aluminiumoxid, Zirkonoxid, Magnesiumoxid, Siliciumdioxid, gemischte Oxide aus solchen Oxiden, Alumosilikate und Zeolithe genannt. Ganz besonders bevorzugt die Erfindung Träger aus Aluminiumoxid und Siliciumdioxid.

Das zu bindende Enzym kann eine Oxidoreduktase, Tansferase, Hydrogenase, Lyase, Ligase oder Isomerase sein. Als Enzyme kommen für das erfindungsgemäße Verfahren insbesondere solche in Betracht, die in industriellen Verfahren eingesetzt werden wie beispielsweise Glucoamylase, Glucoseoxidase, Lactase, Trypsin, Pepsin, Papain, andere Proteasen, Lipase, Pektinase, Invertase, Fumarase, Aspartase, Urease, Aminosäureoxidase, Penicillinacylase, Glucoseisomerase. Besonders bevorzugt ist die Glucoseisomerase.

Die nachfolgenden Beispiele dienen der weiteren Erläuterung der Erfindung.

### Herstellung der Ausgangs- bzw. Vergleichspäparate

Unter Benutzung der Lehre gemäß der DE-2726188-C2, insbesondere hinsichtlich der Bestimmung der optimalen Konzentration der Enzymlösung, wurde aus einem Träger aus SiO₂ mit einer Korngröße von 0,1 bis 0,2 mm und einem häufigsten Porendurchmesser von 430 Å ein trägergebundenes Glucoseisomerasepräparat hergestellt und gemäß dem einen Teilaspekt der Erfindung das Enzym zusätzlich mit Glutardialdehyd vernetzt.

Zur Vernetzung des Enzyms wurde das Präparat 30 Minuten lang mit glutardialdehydhaltigem Fixierungspuffer (50 mM Phosphatpuffer, pH 6, der 0,4 mM MgSO₄ enthielt) behandelt, dem soviel einer höherkonzentrierten Glutardialdehydlösung zugefügt worden war, daß er in Bezug auf Glutardialdehyd eine Konzentration (w/v) von 1 % aufwies. Die Menge des eingesetzten Glutardialdehyd betrug 40 mg/g Katalysator-Trockensubstanz. Nach Waschen des vernetzten Präparats mit glutardialdehydfreiem Fixierungspuffer wurde das Präparat bis zur weiteren Verwendung in Lagerpuffer (50 mM Phosphatpuffer, pH 6, der 0,4 mM MgSO₄ und insgesamt 2000 ppm para-Hydroxybenzoesäure-Ester enthielt) aufbewahrt. Dieses Präparat wird nachfolgend "Standard 1"-Präparat genannt.

In analoger Weise wurde aus einem Träger aus Aluminiumoxid mit einer Korngröße von 0,3 bis 0,6 mm und einem häufigsten Porendurchmesser von 340 Å ein trägergebundenes Glucoseisomerasepräparat hergestellt und das Enzym mit Glutardialdehyd vernetzt. Diese Präparat wird nachfolgend "Standard 2"-Präparat genannt. Auch dieses Präparat wurde bis zur weiteren Verwendung in Lagerpuffer der vorstehend beschriebenen Art aufbewahrt.

### Beispiel 1

Wie für das "Standard 1"-Präparat unter "Herstellung der Ausgangs- und Vergleichspräparate" beschrieben, wurden Präparate hergestellt, die vor der Glutardialdehyd-Vernetzung mit einer 0,5 %igen (w/v) wässrigen Lösung von Polyethyleniminen (Herkunft siehe Liste 1), pH 5, behandelt wurden. Dazu wurden die feuchten Präparate mit 3 ml der 0,5 %igen Polyethyleniminlösung pro Gramm Katalysator-Trockensubstanz überschichtet. Die Mischung wurde unter gelegentlichem Umrühren 30 Minuten bei Raumtemperatur inkubiert. Danach wurde die überstehende Lösung abgesaugt und der Rückstand mit Fixierungspuffer gewaschen. Anschließend wurde die Glutardialdehyd-Vernetzung wie oben bereits beschrieben durchgeführt und die Präparate in Lagerpuffer aufbewahrt.

### Liste 1

| Untersuchte Polyethylenimine | | |
|---|---|---|
| Typ | Hersteller | Präparate-Nr. |
| 7000 | BASF | Beispiel 1/1 |
| 20000 | BASF | 1/2 |
| 80000 | BASF | 1/3 |
| Sedipur VP/FD | BASF | 1/4 |
| Sedipur VP/mf | BASF | 1/5 |
| Sedipur VP/ST | BASF | 1/6 |
| Sedipur VP/P | BASF | 1/7 |
| Polymin P | BASF | 1/8 |
| Epomin-SP-006 | Sumitomo | 1/9 |
| Epomin-SP-018 | Sumitomo | 1/10 |
| Epomin-SP-200 | Sumitomo | 1/11 |
| Epomin-SP-1000 | Sumitomo | 1/12 |

### Beispiel 2

5 g "Standard 1"-Präparat wurden mit 15 ml einer 0,5 %igen (w/v) Polyethyleniminlösung (Polymin P, BASF), pH 5, überschichtet und 30 Minuten bei Raumtemperatur unter gelegentlichem Umrühren inkubiert. Nach Waschen mit Fixierungspuffer wurde das Präparat in Lagerpuffer aufbewahrt.

### Beispiel 3

Analog Beispiel 2 wurde das "Standard 2"-Präparat mit Polyethylenimin modifiziert.

### Beispiel 4

Analog Beispiel 1 wurde ein Präparat mit 2 %iger Polyethyleniminlösung (Polyimin P, BASF) hergestellt.

### Versuch 1

Zum Nachweis der erhöhten Stabilität der erfindungsgemäß hergestellten trägergebundenen Enzyme wurden die im Beispiel 1 hergestellten Präparate mit dem "Standard 1"-Präparat verglichen.

Dazu wurden je 1 ml Präparat in 10 cm lange Säulen mit Temperiermantel gefüllt. Die Säulen wurden anschließend mit Substratlösung beaufschlagt. Als Substratlösung dient 45 %ige (w/v) Glucoselösung, die auf einen pH von 7,5 eingestellt war und als Kofaktoren 120 ppm Mg⁺⁺ und 200 ppm SO₂ in Form von Na₂SO₃ enthielt. Die Substratlösung wurde auf 75 °C vorgewärmt und mit konstantem Durchfluß (Volumengeschwindigkeit 35 v/vh) über die Säule geleitet. Als Maß für die Aktivität der Präparate wurde in regelmäßigen Zeitabständen im abfließenden Substrat die unter dem Einfluß der trägergebundenen Glucoseisomerase aus der Glucose entstandene Fructose polarimetrisch bestimmt. Der sich daraus ergebende Isomerisierungsgrad wurde in einfach logarithmischem Papier gegen die Zeit aufgetragen. Als Maß für die Stabilität diente der lineare Bereich der Aktivitätsabnahme über der Zeit, ausgedrückt in Abnahme des Isomerisierungsgrades [ % ] / 100 h.

Dabei ist die Stabilität umso größer, je kleiner die Aktivitätsabnahme in den Zeitintervall ist. Die erhaltenen Werte sind in Tabelle 1 eingetragen.

Die für die Standard-Präparate angegebenen Werte wurden mit einem Substrat erhalten, das außer den genannten Kofaktoren noch 1 ppm Co⁺⁺ enthielt.

**Tabelle 1**

| Stabilisierungseffekt verschiedener Polyethelyimine | |
|---|---|
| Präparat | Abnahme des Isomerisierungsgrades [ % ] / 100 h |
| Beispiel 1/1 | 5,1 |
| 1/2 | 7,0 |
| 1/3 | 8,8 |
| 1/4 | 6,7 |
| 1/5 | 6,6 |
| 1/6 | 7,2 |
| 1/7 | 7,3 |
| 1/8 | 7,0 |
| 1/9 | 9,2 |
| 1/10 | 6,1 |
| 1/11 | 7,7 |
| 1/12 | 7,3 |
| Standard 1 | 10,7 |

### Versuch 2

Zum Vergleich wurde Versuch 1 mit einigen Präparaten unter veränderten Bedingungen (70 °C, 30,5 v/vh) wiederholt. Das Ergebnis gibt Tabelle 2 wieder.

**Tabelle 2**

| Stabilisierungseffekt verschiedener Polyethylenimine | |
|---|---|
| Präparat | Abnahme des Isomerisierungsgrades [ % ] / 100 h |
| Beispiel 1/8 | 3,5 |
| 1/9 | 3,9 |
| 1/12 | 2,9 |
| Beispiel 2 | 4,3 |
| Standard 1 | 4,8 |

### Versuch 3

Zur Simulation betriebsähnlicher Bedingungen wurde Versuch 1 bei 60 °C wiederholt, jedoch wurde nicht mit konstantem Durchfluß gefahren, es wurde vielmehr der Durchfluß (die Volumengeschwindigkeit) in Abhängigkeit von der Aktivität auf einen konstanten Isomerisierungsgrad von 46,5 % eingestellt. Als Maß für die Stabilität wurde die Halbwertszeit, gemessen in Stunden, ermittelt. Die erhaltenen Werte sind in Tabelle 3 eingetragen. In den Versuch 3 wurde auch das "Standard 2"-Präparat und das Präparat gemäß Beispiel 3 einbezogen.

**Tabelle 3**

| Halbwertszeiten verschiedener Präparate unter betriebsähnlichen Bedingungen | |
|---|---|
| Präparat | Halbwertszeit * [ h ] |
| Standard 1 | 1260 |
| Beispiel 1/8 | 2150 |
| Beispiel 2 | 2200 |
| Standard 2 | 1900 |
| Beispiel 3 | 2500 |

| | |
|---|---|
| * Die Halbwertszeit wurde bestimmt aus dem Diagramm In Aktivität → t, wobei A durch Extrapolieren erhalten wurde. | |

### Versuch 4

Zum Nachweis dafür, daß die erfindungsgemäß hergestellten trägergebundenen Enzyme nicht nur eine bessere Zeitstabilität, sondern auch eine bessere Temperaturstabilität aufweisen, wurde der Versuch 1 mit dem "Standard 1"-Präparat und dem Präparat gemäß Beispiel 4 wiederholt mit der Maßgabe, daß die Substratlösung auf 70 °C, 75 °C und 80 °C vorerwärmt und dazu entsprechend angepaßt der Durchfluß auf Volumengeschwindigkeiten von 30,5 v/vh, 35 v/vh und 41 v/vh eingestellt wurde. Als Maß für die Stabilität wurde wie in Versuch 1 die Aktivitätsabnahme pro 100 Stunden, ausgedrückt in % Isomerisierungsgrad, herangezogen. Die erhaltenen Werte sind in Tabelle 4 eingetragen.

**Tabelle 4**

| Temperatur [ °C ] | Abnahme des Isomerisierungsgrades [ % ] / 100 h | |
|---|---|---|
| | Präparat | |
| | Standard 1 | Beispiel 4 |
| 70 | 4,8 | 2,0 |
| 75 | 10,7 | 6,8 |
| 80 | 60,3 | 42,2 |

## Patentansprüche

1. Verfahren zur Herstellung trägergebundener Enzyme, beim dem man einen Träger, der zur Ausbildung von kovalenten Bindungen befähigte funktionelle Gruppen aufweist, mit einem Enzym belegt, dadurch gekennzeichnet, daß man das erhaltene trägergebundene Enzym zusätzlich in beliebiger Reihenfolge mit einem bifunktionellen Vernetzungsmittel und einem Polyamin behandelt.

2. Verfahren nach Anspruch 1, dadurch gekennzeichnet, daß man das trägergebundene Enzym zunächst mit dem Polyamin und anschließend mit dem Vernetzungsmittel behandelt.

3. Verfahren nach Anspruch 1 oder 2, dadurch gekennzeichnet, daß man als bifunktionelles Vernetzungsmittel Glutardialdehyd verwendet.

4. Verfahren nach Anspruch 3, dadurch gekennzeichnet, daß man mit 5 bis mg Glutardialdehyd, vorzugsweise mit 25 bis 250 mg Glutardialdehyd, pro Gramm Katalysator-Trockensubstanz behandelt.

5. Verfahren nach Anspruch 3 oder 4, dadurch gekennzeichnet, daß man Glutardialdehyd in 0,1 bis 10 %iger (w/v) wässriger Lösung, vorzugsweise in 0,5 bis 5 %iger (w/v) wässriger Lösung, verwendet.

6. Verfahren nach einem der Ansprüche 3 bis 5, dadurch gekennzeichnet, daß man die Behandlung mit Glutardialdehyd bei einem pH zwischen 4 und 9, vorzugsweise bei einem pH zwischen 5 und 8, durchführt.

7. Verfahren nach einem der Ansprüche 3 bis 6, dadurch gekennzeichnet, daß man die Behandlung mit Glutardialdehyd 15 bis 120 Minuten lang, vorzugsweise 30 bis 60 Minuten lang, durchführt.

8. Verfahren nach einem der Ansprüche 1 oder 2, dadurch gekennzeichnet, daß man als Polyamin Polyethylenimin verwendet.

9. Verfahren nach Anspruch 8, dadurch gekennzeichnet, daß man Polyethylenimin mit einem Molekulargewicht von bis zu 2.000.000 Dalton verwendet.

10. Verfahren nach Anspruch 9, dadurch gekennzeichnet, daß man Polyethylenimin mit einem Molekulargewicht zwischen 600 und 1.000.000 Dalton verwendet.

11. Verfahren nach einem der Ansprüche 8 bis 10, dadurch gekennzeichnet, daß man mit 3 bis 600 mg Polyethylenimin, vorzugsweie mit 15 bis 240 mg Polyethylenimin, pro Gramm Katalysator-Trockensubstanz behandelt.

12. Verfahren nach einem der Ansprüche 8 bis 11, dadurch gekennzeichnet, daß man Polyethylenimin in 0,1 bis 20 %iger (w/v) wäßriger Lösung, vorzugsweise in 0,5 bis 8 %iger (w/v) wäßriger Lösung, verwendet.

13. Verfahren nach einem der Ansprüche 8 bis 12, dadurch gekennzeichnet, daß man die Behandlung mit Polyethylenimin bei einem pH zwischen 4 und 9, vorzugsweise bei einem pH zwischen 5 und 8, durchführt.

14. Verfahren nach einem der Ansprüche 8 bis 13, dadurch gekennzeichnet, daß man die Behandlung mit Polyethylenimin wenigstens 5 Minuten lang, vorzugsweise wenigstens 15 Minuten lang, durchführt.

15. Verfahren nach einem der vorhergehenden Ansprüche, dadurch gekennzeichnet, daß man einen porösen anorganischen Träger auf der Basis Aluminiumoxid oder Kieselsäure, verwendet.

16. Verfahren nach einem der vorhergehenden Ansprüche, dadurch gekennzeichnet, daß man als Enzym Glucoseisomerase verwendet.

## Claims

1. Method for producing carrier-bound enzymes, in which a carrier which has functional groups capable of forming covalent bonds is coated with an enzyme, characterised in that the resulting carrier-bound enzyme is additionally treated in any sequence with a bifunctional cross-linking agent and a polyamine.

2. Method according to Claim 1, characterised in that the carrier-bound enzyme is first treated with the polyamine and then with the cross-linking agent.

3. Method according to Claims 1 or 2, characterised in that glutaric dialdehyde is used as a bifunctional cross-linking agent.

4. Method according to Claim 3, characterised in that treatment is carried out with 5 to 500 mg glutaric dialdehyde, preferably with 25 to 250 mg glutaric dialdehyde, per gramme dry catalyst substance.

5. Method according to Claim 3 or 4, characterised in that glutaric dialdehyde in a 0.1 to 10% (w/v) aqueous solution, preferably in a 0.5 to 5% (w/v) aqueous solution, is used.

6. Method according to one of Claims 3 to 5, characterised in that the treatment with glutaric dialdehyde takes place at a pH between 4 and 9, preferably at a pH between 5 and 8.

7. Method according to one of Claims 3 to 6, characterised in that the treatment with glutaric dialdehyde is carried out for 15 to 120 minutes, preferably 30 to 60 minutes.

8. Method according to one of Claims 1 or 2, characterised in that polyethylene imine is used as polyamine.

9. Method according to Claim 8, characterised in that polyethylene imine with a molecular weight of up to 2,000,000 Dalton is used.

10. Method according to Claim 9, characterised in that polyethylene imine with a molecular weight of between 600 and 1,000,000 Dalton is used.

11. Method according to one of Claims 8 to 10, characterised in that treatment is carried out with 3 to 600 mg polyethylene imine, preferably with 15 to 240 mg polyethylene imine, per gramme dry catalyst substance.

12. Method according to one of Claims 8 to 11, characterised in that polyethylene imine in a 0.1 to 20% (w/v) aqueous solution, preferably in 0.5 to 8% (w/v) aqueous solution is used.

13. Method according to one of Claims 8 to 12, characterised in that the treatment with polyethylene imine is carried out at a pH between 4 and 9, preferably at a pH between 5 and 8.

14. Method according to one of Claims 8 to 13, characterised in that the treatment with polyethylene imine is carried out for at least 5 minutes, preferably at least 15 minutes.

15. Method according to one of the preceding Claims, characterised in that a porous inorganic carrier based on aluminium oxide or silicic acid is used.

16. Method according to one of the preceding Claims, characterized in that glucose isomerase is used as an enzyme.

## Revendications

1. Procédé de préparation d'enzymes fixées sur un support dans lequel on recouvre un support comportant des groupes fonctionnels aptes à former des liaisons covalentes d'une enzyme, caractérisé en ce que l'on traite en outre l'enzyme fixée sur un support obtenue, dans un ordre quelconque avec un réticulant bifonctionnel et une polyamine.

2. Procédé selon la revendication 1, caractérisé en ce que l'on traite l'enzyme fixée sur un support tout d'abord par la polyamine, puis par le réticulant.

3. Procédé selon la revendication 1 ou 2, caractérisé en ce qu'on emploie du glutaraldéhyde en tant que réticulant bifonctionnel.

4. Procédé selon la revendication 3, caractérisé en ce que l'on traite avec 5 à 500 mg de glutaraldéhyde, de préférence avec 25 à 250 mg de glutaraldéhyde par gramme de substance sèche de catalyseur.

5. Procédé selon la revendication 3 ou 4, caractérisé en ce qu'on utilise le glutaraldéhyde en solution aqueuse de 0,1 à 10% (poids/vol.), de préférence en solution aqueuse de 0,5 à 5% (poids/vol.).

6. Procédé selon l'une des revendications 3 à 5, caractérisé en ce qu'on effectue le traitement avec du glutaraldéhyde à un pH entre 4 et 9, de préférence à un pH entre 5 et 8.

7. Procédé selon l'une des revendications 3 à 6, caractérisé en ce qu'on effectue le traitement avec du glutaraldéhyde pendant 15 à 120 minutes, de préférence pendant 30 à 60 minutes.

8. Procédé selon l'une des revendications 1 ou 2, caractérisé en ce qu'on emploie la polyéthylèneimine en tant que polyamine.

9. Procédé selon la revendication 8, caractérisé en ce qu'on emploie une polyéthylèneimine ayant un poids moléculaire jusqu'à 2.000.000 daltons.

10. Procédé selon la revendication 9, caractérisé en ce qu'on emploie une polyéthylèneimine ayant un poids moléculaire entre 600 et 1.000.000 daltons.

11. Procédé selon l'une des revendications 8 à 10, caractérisé en ce que l'on traite avec 3 à 600 mg de polyéthylèneimine, de préférence avec 15 à 240 mg de polyéthylèneimine par gramme de substance sèche de catalyseur.

12. Procédé selon l'une des revendications 8 à 11, caractérisé en ce qu'on utilise la polyéthylèneimine en solution aqueuse de 0,1 à 20% (poids/vol.), de préférence en solution aqueuse de 0,5 à 8% (poids/vol.).

13. Procédé selon l'une des revendications 8 à 12, caractérisé en ce qu'on effectue le traitement avec la polyéthylèneimine à un pH entre 4 et 9, de préférence à un pH entre 5 et 8.

14. Procédé selon l'une des revendications 8 à 13, caractérisé en ce qu'on effectue le traitement avec la polyéthylèneimine pendant au moins 5 minutes, de préférence pendant au moins 15 minutes.

15. Procédé selon l'une des revendications précedentes, caractérisé en ce qu'on emploie un support minéral poreux à base d'oxyde d'aluminium ou d'acide silicique.

16. Procédé selon l'une des revendications précedentes, caractérisé en ce qu'on utilise la glucose-isomérase en tant qu'enzyme.
